# EUROPÄISCHE PATENTSCHRIFT

(11) **EP 1 491 161 B1**
(45) Veröffentlichungstag und Bekanntmachung des Hinweises auf die Patenterteilung: **15.04.2015**
(21) Anmeldenummer: 04011398.7
(22) Anmeldetag: 13.05.2004
(51) Int. Cl.: A61F 2/91, A61F 2/915

(54) **Biegsame Welle**
Flexible shaft
Arbre flexible

(30) Priorität: 26.06.2003 DE 10328882
(43) Veröffentlichungstag der Anmeldung: 29.12.2004
(73) Patentinhaber: Admedes Schuessler GmbH, 75179 Pforzheim (DE)
(72) Erfinder: Ehrlinspiel, Michael, 76256 Weingarten (DE); Flaxmeier, Erik, 76307 Karlsbad (DE)
(74) Vertreter: Müller-Boré & Partner Patentanwälte PartG mbB

(56) Entgegenhaltungen:
- WO-A-99/44543
- US-A1- 2003 105 511

## Beschreibung

Die Erfindung betrifft eine biegsame Welle zum Einführen in einen Kanal eines lebenden Körper sowie deren bevorzugte Verwendung, mit einer Vielzahl von aneinander angrenzenden Zellen, deren Wände je aus Wandabschnitten ausgebildet sind. Insbesondere betrifft die Erfindung eine biegsame Welle, die zum Einführen in ein Lumen bzw. einen Kanal mit unterschiedlichen Eigenschaften geeignet ist. Solche unterschiedlichen Eigenschaften von Lumen können verschiedene Krümmungen, Seitenverzweigungen, variable Durchmesser oder verschieden nachgiebige Lumenwände sein.

Biegsame Wellen der oben genannten Art werden verwendet, um beispielsweise Stents in verschiedene Kanäle lebender Körper, wie z.B. Blutgefäße, Speiseröhre, Harnröhre, Nierengänge, einzuführen. Der Stent muß nach dem Einführen radial expandiert werden, wobei ein Stab bzw. eine Seele verwendet wird, die durch das Innere der biegsamen Welle hindurch geleitet ist. Zu diesem Zweck muß die biegsame Welle schlauchartig gestaltet sein und sich auch gebogenen bzw. kurvigen Kanal- bzw. Aderbereichen anpassen.

WO 99/44543 A beschreibt verbesserte Stentzellenkonficiurationen, bei denen die Zellen drei oder mehr vergrößerte Endabschnitte haben, um von einer gemeinsamen Mitte innerhalb der Zelle strahlenförmig auszugehen.

Aufgabe der Erfindung ist es, eine biegsame Welle zum Einführen in ein Lumen eines lebenden Körpers zu schaffen, die kostengünstig herzustellen ist und dabei zugleich eine hohe Flexibilität gepaart mit einer ausreichenden Stabilität, insbesondere zum Abstützen eines Stabes zum Expandieren eines Stents aufweist. Diese Aufgabe wird erfindungsgemäß durch eine biegsame Welle gemäß Anspruch 1 gelöst. Bevorzugte Ausführungsformen sind Gegenstand der Unteransprüche.

Dementsprechend ist die Aufgabe erfindungsgemäß mit einer eingangs genannten Welle gelöst, bei der bei mindestens einer Zelle im Querschnitt betrachtet die Wand in Umfangsrichtung abwechselnd aus jeweils einem konkaven Wandabschnitt und einem benachbarten konvexen Wandabschnitt ausgebildet ist. Vorteilhafte Weiterbildungen der Erfindung sind in den Unteransprüchen beschrieben.

Bei der erfindungsgemäßen biegsamen Welle ist die Wand in Umfangsrichtung aufeinanderfolgend aus konkaven und konvexen Wandabschnitten ausgebildet. Auf diese Weise sind in der Wand mehrere Bereiche gebildet, an denen beim Biegen bzw. Verformen der biegsamen Welle an jeder Zelle je ein zunächst gefalteter konkaver und ein benachbarter konvexer Wandabschnitt gestreckt bzw. entfaltet werden. Das Strecken der gefalteten konkaven und konvexen Wandabschnitte führt zu einer gleichzeitigen Vergrößerung der einzelnen Zelle an mehreren Bereichen der Wand.

Das gleichzeitige Strecken bzw. Entfalten der Wand an mehreren Bereichen bewirkt, daß während des Biegens bzw. Verformens die Wand der einzelnen Zelle in mehrere Richtungskomponenten, insbesondere mehr als zwei Richtungskomponenten verformt wird. Die erfindungsgemäße Welle wird beim Biegen jedoch nicht in radialer Richtung komprimiert, so daß ein in der biegsamen Welle geführter Stab nicht geklemmt wird.

Bei der erfindungsgemäßen Welle wird beim Biegen bzw. Verformen eher der innere Durchmesser leicht vergrößert, da die sich streckenden Wände von verformten Zellen verstärkt in Richtung auf eine radiale Expansion der Welle drängen.

Erfindungsgemäß pflanzt sich ferner der Streckvorgang der Wände über die Zellen hinweg fort. Die Expansion einer Zelle in der Wand der erfindungsgemäßen biegsamen Welle führt auf diese Weise zu einer gleichmäßigen Verformung in einem größeren Bereich der Wandstruktur. Unerwünschte, lokal begrenzte und daher strukturschwächende Verformungen sind hingegen erfindungsgemäß vermieden.

Bei Wegnahme der Deformationsbelastung an einer erfindungsgemäßen Welle stoppt deren Deformation nahezu schlagartig, ohne daß hohe Eigenspannungen in der Welle verbleiben würden.

Darüber hinaus ist die Wand einer erfindungsgemäßen Welle über den Umfang hinweg verhältnismäßig gleichmäßig gefaltet bzw. gewellt ausgebildet. Eine Beschichtung der Wand, beispielsweise mit einem Gleitmittel ist daher einfacher auszubilden.

Aufgrund der gleichmäßig gewellten Ausformung der Wand sind erfindungsgemäß Spannungsspitzen und lokale Deformationsspitzen in der biegsamen Welle vermieden. Weiterhin werden enge Radien bzw. die Ausbildung unterschiedlicher Radien vorteilhaft vermieden. Dadurch ist die Gefahr eines Beschädigens oder Abplatzens insbesondere einer metallischen oder keramischen Beschichtung der Wände der erfindungsgemäßen Welle vermieden. Ferner wird dadurch die Gefahr einer Materialüberbelastung durch Spannungsspitzen reduziert. Die Materialeigenschaften des jeweils eingesetzten Werkstoffs werden erfindungsgemäß besser ausgenutzt.

Bei einer biegsamen Welle erhöht sich unter bestimmten Bedingungen bei zunehmender Verformung die Festigkeit des Materials der Welle. Dieser sogenannte Verfestigungseffekt kann erfindungsgemäß besonders gut ausgenutzt werden, weil die Wände der Welle gleichmäßig und zugleich in verhältnismäßig geringem Ausmaß verformt werden. Die Verformung zur Verfestigung des Materials kann erfindungsgemäß also sehr genau gesteuert werden und ist darüber hinaus über die gesamte Welle hinweg nahezu gleich ausgeprägt.

Gemäß der Erfindung ist die Wand einer Zelle einer erfindungsgemäßen Welle allein aus gewölbten bzw. gefalteten Wandbereichen und Knotenpunkten ausgebildet, an denen solche Wandbereiche aneinanderstoßen.

Darüber hinaus verkürzt sich die erfindungsgemäße Welle beim Verformen nicht in axialer Richtung. Eine Welle gemäß der Erfindung zeichnet sich vielmehr durch ein gleichmäßiges Aufdehnen der Zellenstruktur in mehrere Richtungen aus.

Die erfindungsgemäße Welle kann ferner besonders homogen expandiert und wieder geschrumpft werden. Dieser Vorteil kann genutzt werden, wenn die Welle im expandierten oder teilexpandierten Zustand gefertigt werden soll. Weiterhin kann dieser Vorteil bei Verfahren der Elektropolitur und der Beschichtung genutzt werden. Die Welle kann vor dem Verfahren gestreckt und nachfolgend wieder gleichmäßig komprimiert werden. Die Qualität der durch das Verfahren erzielten Oberflächen kann auf diese Weise erhöht werden.

Die erfindungsgemäße Vernetzung und Ausbildung unterschiedlicher Deformationsrichtungen ist, wie bereits erwähnt, besonders beim Biegen einer expandierten Welle von Vorteil. Die Zellen einer erfindungsgemäßen Welle verformen sich je nach Lage der Biegelinie gleichmäßig expandierend oder stauchend und ermöglichen so eine homogene Biegung der Welle.

Die Erfindung kann besonders sinnvoll bei Wellen aus rostfreiem Edelstahl, Tantal, Niob, Kobaltlegierungen und anderen Werkstoffen wie z.B. Polymeren, selbstabbaubaren Werkstoffen (z.B. Milchsäure-Werkstoffen bzw. -Derivate), sowie bei Wellen aus Nitinol (Nickel-Titan-Legierungen) und/oder aus anderen selbstexpandierbaren Werkstoffen bzw. Formgedächniswerkstoffen verwendet werden.

Bei einer vorteilhaften Weiterbildung der Erfindung ist im Querschnitt betrachtet mindestens ein konvexer Wandabschnitt mit einem Krümmungsradius ausgebildet, der im wesentlichen gleich einem Krümmungsradius eines benachbarten konkaven Wandabschnitts gestaltet ist. Im wesentlichen gleiche Krümmungsradien an der gefalteten Wand der Zellen führen zu einer besonders gleichmäßigen Struktur der Welle. Eine solche Welle kann daher besonders gut elektrochemisch poliert oder beschichtet werden. Ferner werden Coating- bzw. Beschichtungsvorgänge erleichtert, weil Abschattungseffekte beim sogenannten PVD- oder CVD-Verfahren vermieden bzw. vermindert und/oder Tropfeneffekte bei Tauchverfahren (die durch Kapillarwirkung enger Radien zustandekommen) vermieden werden.

Die genannten Vorteile einer erfindungsgemäßen biegsamen Welle können weiter vorteilhaft ausgeprägt werden, indem im Querschnitt betrachtet die Wandabschnitte jeweils gleichartig oder fast gleichartig ausgebildet sind. Eine derartige weitergebildete Welle ist bis auf Knotenpunkte insgesamt aus gleichen Geometrieelementen aufgebaut. Die Geometrieelemente können identische Außen- und Innendurchmesser und identische Strukturbreiten aufweisen. Aus solchen Geometrielementen ist durch verschiedenartige Verschachtelung ein Basiselement in Gestalt einer Zelle gebildet.

Darüber hinaus können im Querschnitt betrachtet die Wandabschnitt vorteilhaft jeweils kreisbogenförmig bzw. als Kreisbogen- bzw. Kreisstruktur ausgestaltet sein. Spannungsspitzen im Material der Welle können auf diese Art und Weise vermieden werden und eine Torsion der Welle wird erleichtert.

Ferner ist es vorteilhaft, wenn im Querschnitt betrachtet mindestens eine Zelle je aus mindestens drei konvexen Wandabschnitten gestaltet ist, bei denen jeweils zwischen zwei konvexen Wandabschnitten ein konkaver Wandabschnitt ausgebildet ist. Eine derartige einzelne Zelle einer erfindungsgemäßen Welle wird beim Expandieren in drei zueinander nicht parallelen Richtungen gestreckt. Die Richtungen sind insbesondere jeweils um einen Winkel von 120° versetzt. Die erfindungsgemäße biegsame Welle besitzt damit eine mehrachsige Deformationskomponente und wird im Gegensatz zu bekannten Wellen, die nur zwei um einen Winkel von 90° versetzte Richtungskomponenten aufweisen, in besonders einfacher Weise gleichmäßig expandiert, wie oben bereits erläutert wurde.

Die erfindungsgemäße Welle kann besonders klein komprimiert und zugleich leicht und gleichmäßig verformt werden, indem im Querschnitt betrachtet bei komprimiertem Zustand der Welle die konvexen Wandabschnitte mindestens einer Zelle aneinander anliegend ausgebildet sind.

Gemäß der Erfindung können darüber hinaus die oben als vorteilhaft beschriebenen und in den Unteransprüchen dargelegten Weiterbildungen der Erfindung auch ohne die im Anspruch 1 genannten Merkmale als einzelne Gegenstände oder in Kombination untereinander selbständig schutzwürdig sein.

Es sei noch angemerkt, daß die einzelnen konkaven und konvexen Wandabschnitte gemäß der Erfindung durch verschiedenartige Ausformungen gebildet sein können. Diese Ausformungen können im Querschnitt kreisbogenförmig sein oder insbesondere kurze gerade Abschnitte aufweisen. Grundgedanke ist es, daß die Wand einer Zelle der biegsamen Welle durch eine Aneinanderreihung von konkaven und konvexen Wandabschnitten gebildet ist, die insbesondere gleichartig ausgebildet sein können. Die einzelnen Wandabschnitte können vorteilhaft aus ein und demselben geometrischen Grundelement aufgebaut sein. Diese Grundelemente können durch Knotenpunkte verbunden sein, deren äußere Gestalt insbesondere vorteilhaft wiederum an die äußere Form eines Grundelements angepaßt ist.

Die derart gestaltete biegsame Welle ist sowohl hinsichtlich ihrer erzielbaren Oberflächenqualität und damit auch ihrer Biokompatibilität als auch der erforderlichen Fertigungstechnik besonders vorteilhaft. Zur Erzielung einer besonders guten Biokompatibilität wird die Welle in der Regel einer Oberflächenveredelung, wie etwa einer elektrochemischen Politur, unterzogen, welche bevorzugt einen dünnen, schützenden Oxidfilm auf der Oberfläche produziert. Ein solches Verfahren beruht auf einem gezielten Masseabtrag der Oberfläche der Welle. Die elektropolierte Oberfläche besitzt sehr geringe Rauhigkeit und eine hohe Reinheit. Das Polierverfahren wird stromunterstützt in einem Säuregemisch durchgeführt, wobei für eine gleichmäßige Politur, feldbedingt, eine möglichst homogene Bauteilstruktur vorhanden sein muß. Die erfindungsgemäße Welle besteht, wie oben erläutert worden ist, aus einer solchen, besonders homogenen Struktur. Besonders große oder kleine Radien an der Struktur sind erfindungsgemäß vermieden. Bekannte Wellen bestehen hingegen aus unterschiedlichen konstruktiven Komponenten, die lediglich hinsichtlich der mechanischen Eigenschaften der jeweiligen Welle optimiert wurden.

Gemäß der Erfindung wird eine Verwendung einer biegsamen Welle nach der Erfindung oder einer bevorzugt Ausführungsform hiervon bereitgestellt zum Einführen zum Einführen bzw. Anordnen von einer Spreizstruktur, einer Metallelektrode und/oder einer Endoprothesen bzw. Stent in ein Hohlorgan bzw. Lumen bzw. Kanal bzw. Hohlraum.

Weiterhin kann die biegsame Welle in einer vorteilhaften Ausführungsform ebenfalls an einem Führungsdrahtkörper verwendet werden. Hierbei bildet die biegsame Welle eine hochflexible Spitze bzw. ein hochflexibles distales Ende des Führungsdrahts aus. Somit kann eine optimale Zuführbarkeit ins Zielgebiet sichergestellt werde, da die biegsame Wellenstruktur, d. h. die biegsame Welle, stufenlos und ohne Einschränkungen ähnlich eines gummielastischen Schlauchs abgebogen bzw. abgewinkelt werden kann. Insbesondere kann die biegsame Welle eine hohe Torsionssteifigkeit aufweisen, so dass diese leichter in eng gewundene und kleine Gefäße eingeführt werden kann. Dies ist insbesondere vorteilhaft beim Sondieren von neurovaskulären Blutgefäßen, wie beispielsweise der Aorta vertebralis, der Aorta cerebralis oder der Aorta carotis.

Besonders vorteilhafterweise kann die biegsame Welle als gelenkfreier Körper bzw. als gelenkfreies Ende in einem steuerbaren Endoskopkopf verwendet werden. Da durch die zusammenhängenden Wandabschnitte die Welle ihre Biegsamkeit erhält, sind zusätzliche Verbindungselemente in der Wellenstruktur, wie Kontaktstifte etc. auf, welche gegenüber dem Körper der biegsamen Welle eine Relativbewegung ausführen, nicht notwendig. Hierdurch lässt sich vorteilhafterweise ein im wesentlichen wartungsfreies Bauteil schaffen, welches in einem Endoskop eingesetzt werden kann.

In einer weiteren Ausführungsform könnte die biegsame Welle eine rotierende bzw. rotierbare Antriebswelle ausbilden. Somit wird eine Antriebswelle vorgesehen, welche eine mehrachsige Bewegungsfreiheit aufweist, also in unidirektionaler weise beweglich ist. Hierbei ist eine stufenlose Biegung bzw. Abwinklung möglich, wobei zusätzlich eine Rotationsbewegung zwischen zwei achsversetzten Punkten übertragen werden kann. Somit kann vorteilhafterweise wird eine achsversatz-kompensierende flexible Antriebswelle bzw. -kupplung vorgeschlagen werden.

Nachfolgend werden Ausführungsbeispiele einer erfindungsgemäßen Welle anhand der beigefügten schematischen Zeichnungen näher erläutert. Es zeigen:
- Fig. 1a bis 1d: eine Abfolge von Graphiken zur Veranschaulichung des Aufbaus einer Zelle eines ersten Ausführungsbeispiels einer erfindungsgemäßen biegsamen Welle,
- Fig. 2a bis 2c: eine Abfolge von Graphiken zur Veranschaulichung des Aufbaus einer Struktur aus Zellen des ersten Ausführungsbeispiels einer erfindungsgemäßen biegsamen Welle,
- Fig. 3: einen Querschnitt des ersten Ausführungsbeispiels einer erfindungsgemäßen biegsamen Welle an einem komprimierten Strukturabschnitt,
- Fig. 4: einen Querschnitt des ersten Ausführungsbeispiels einer erfindungsgemäßen biegsamen Welle an einem expandierten Strukturabschnitt,
- Fig. 5: einen Querschnitt eines zweiten Ausführungsbeispiels einer erfindungsgemäßen biegsamen Welle an einem komprimierten Strukturabschnitt, und
- Fig. 6: eine perspektivische Ansicht des ersten Ausführungsbeispiels einer erfindungsgemäßen biegsamen Welle im nicht verformten bzw. komprimierten Zustand.

Gemäß einer bevorzugten Ausführungsform der Erfindung weist eine biegsame Welle bzw. Biegewelle eine Vielzahl von zumindest bereichsweise angeordneten Zellen 26, welche eine zumindest lokale Biegung bzw. Verformung der biegsamen Welle z.B. zur Anpassung an eine Form eines Körper-Lumens bzw. -Kanals ermöglicht. Bei einer Biegung bzw. Verformung der biegsamen Welle werden ein oder mehrere Zellen 26 expandiert und/oder ein oder mehrere Zellen 26 komprimiert, so daß im Ergebnis eine Biegung bzw. Verformung der biegsamen Welle möglich ist.

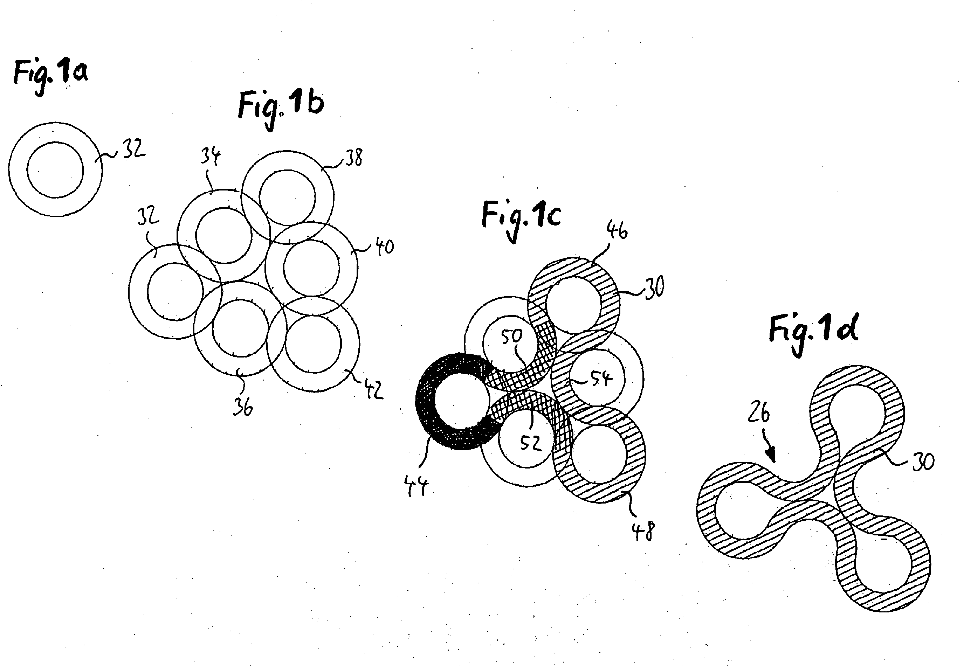

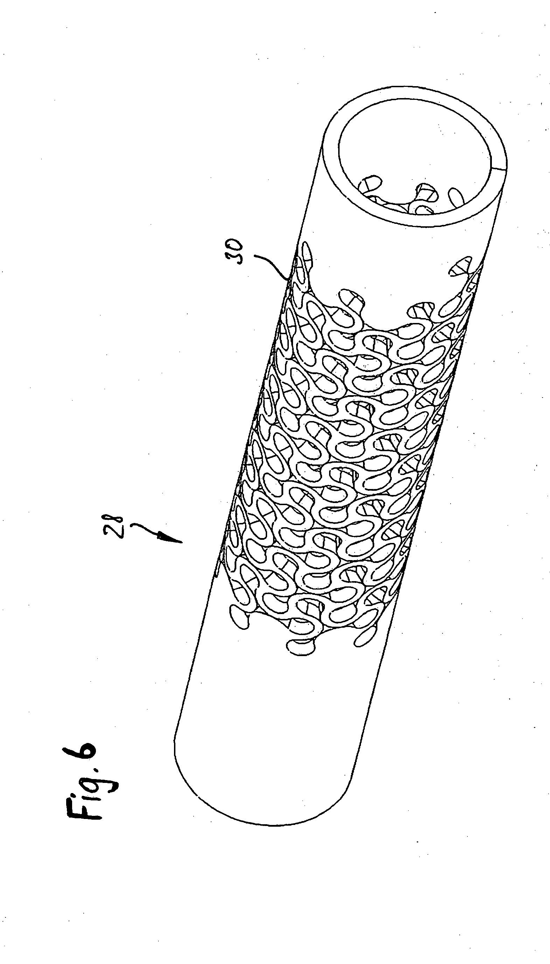

In den Fig. 1a bis 1d ist der Grundaufbau einer bevorzugten Zelle 26 (siehe Fig. 1d) veranschaulicht, die kombiniert mit weiteren Zellen einen biegsamen Bereich zwischen zwei nicht biegsamen Endbereichen eine biegsame Welle 28 (siehe Fig. 6) bildet. Die Zelle 26 weist eine Wand 30 auf, die aus einzelnen Wandabschnitten zusammengesetzt ist.

Als Grundelement für die einzelnen Wandabschnitte dient jeweils ein Kreisring 32, wie er in Fig. 1a veranschaulicht ist.

An einen solchen Kreisring 32 sind gedanklich zwei weitere Kreisringe 34 und 36 angelegt. Die Wände der angelegten Kreisringe 34 und 36 berühren sich und überdecken jeweils die Wand des ersten Kreisrings 32.

An die Kreisringe 34 und 36 sind wiederum gedanklich drei Kreisringe 38, 40 und 42 angelegt. Die Wand des Kreisrings 38 überdeckt die Wände der Kreisringe 34 und 40. Die Wand des Kreisrings 40 überdeckt die Wände der Kreisringe 38 und 42 und berührt die Wände der Kreisringe 34 und 36. Die Wand des Kreisrings 42 überdeckt die Wände der Kreisringe 36 und 40.

Insgesamt sind auf diese Weise sechs Kreisringe zu einem Dreieck zusammengesetzt, wobei sich die Wände der Kreisringe im äußeren Bereich des Dreiecks jeweils überdecken und sich im Zentralbereich des Dreiecks jeweils berühren (siehe Fig. 1b).

In Fig. 1c ist dargestellt, wie aus den derart angeordneten Kreisringen 32 bis 42 die Wandabschnitte der Wand 30 einer einzelnen Zelle 26 aufgebaut sind.

Die Wand 30 ist grundsätzlich im Querschnitt betrachtet schlangenlinienförmig aus jeweils konkaven und konvexen Wandabschnitten gestaltet, die sich in Umfangsrichtung der Wand 30 abwechseln bzw. alternierend angeordnet sind und nachfolgend genauer erläutert werden.

Gemäß der Grundstruktur des Dreiecks der Fig. 1b weist auch die Wand 30 drei "Eckbereiche" auf, die jeweils aus einem konkaven Wandabschnitt 44, 46 und 48 gebildet sind. Die Wandabschnitte 44, 46 und 48 sind bevorzugt kreisbogenförmig und stellen jeweils einen Teil der Kreisringe 32, 38 bzw. 42 gemäß Fig. 1b dar.

Zwischen den konkaven Wandabschnitten 44, 46 und 48 sind jeweils konvexe Wandabschnitte 50, 52 und 54 ausgebildet, die von den Kreisringen 34, 36 bzw. 40 stammen. Von diesen Wandabschnitten berühren sich bevorzugt jeweils die Wandabschnitte 50 und 52, 52 und 54 sowie 50 und 54 im vollständig komprimierten Zustand einer Zelle 30, wie er in Fig. 1c dargestellt ist. Im komprimierten Zustand einer Zelle 30 müssen sich die Wandabschnitte 50, 52 und 54 aber nicht zwingend berühren. Es sind auch Zustände möglich, bei denen die Zelle 30 nur teilweise komprimiert ist und die Wandabschnitte 50, 52 und 54 zumindest geringfügig beabstandet sind.

Demnach ist die "gefaltete" Wand 30 einer Zelle 26, wie sie in Fig. 1d veranschaulicht ist, insgesamt aus den konkaven bzw. konvexen Wandabschnitten 44 bis 54 zusammengesetzt.

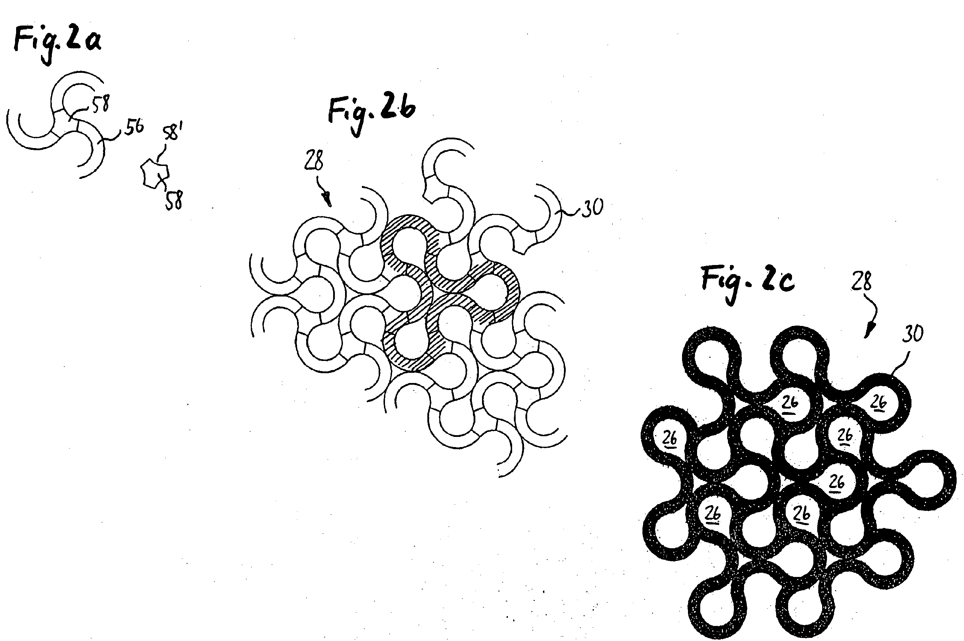

Die biegsame Welle 28 ist aus einer Vielzahl Zellen 26 aufgebaut, die aneinander anliegen. Die in Fig. 1d veranschaulichte Zelle 26 findet sich so in einer biegsamen Welle 28 wieder, wie sie in Fig. 2b und Fig. 6 gezeigt ist.

Aufgrund der Kombination der Zellen 26 zu einer biegsamen Wellenstruktur gehören die einzelnen Wandabschnitte 44 bis 54 einer Zelle 26 auch zugleich als Wandabschnitte zu benachbarten Zellen. Dieser Umstand ist in den Fig. 2a bis 2c veranschaulicht.

Die Graphik gemäß Fig. 2b zeigt, daß die Wände 30 einer biegsamen Welle 28 grundsätzlich auch als strukturierter Aufbau aus vielen Kreisbögen 56 betrachtet werden kann, die zwischen Knoten bzw. Verbindungselementen 58 angeordnet sind. Von einem Knoten 58 stehen dabei jeweils drei Kreisbögen 56 ab.

Der einzelne Kreisbogen 56 erstreckt sich über einen Winkelbereich zwischen 160° und 180°, insbesondere über einen Winkelbereich von etwa 170°. Er ist an seiner Innenseite und seiner Außenseite jeweils im Querschnitt kreisförmig gestaltet, so daß seine Oberflächen Teile von Kreiszylinderflächen darstellen. Die Wand des einzelnen Kreisbogens 56 weist bevorzugt eine konstante Dicke auf. Die einzelnen Knoten 58 verbinden jeweils drei solcher Kreisbögen 56.

Die Knoten 58 weisen jeweils drei Seiten bzw. Endbereiche auf, an denen jeweils ein Kreisbogen 56 anschließt. Zwischen den drei Endbereichen ist an den Knoten 58 jeweils eine konkave Rundung 58' ausgebildet, deren Radius mit dem inneren Radius eines Kreisbogens 56 im wesentlichen übereinstimmt. Die Rundungen der Kreisbögen 56 gehen also glatt in die benachbarten Rundungen 58' von Knoten 58 über, an die sich wiederum Kreisbögen 56 anschließen. Die Krümmung der Oberfläche am Übergang ist konstant.

Die Rundungen 58' der Knoten 58 und die inneren Oberflächen der Kreisbögen 56 gehören zu den oben beschriebenen konkaven Wandbereichen 50, 52 und 54. Die konvexen Wandabschnitte 44, 46 oder 48 werden von den äußeren Oberflächen der Kreisbögen 56 gebildet. An den Übergängen zwischen Kreisbögen 56 und Knoten 58 sind daher jeweils dort Wendepunkte der Krümmung vorhanden, wo an einen Knoten 58 ein konvexer Wandabschnitt 44, 46 oder 48 grenzt, wo also ein konkaver Wandabschnitt in einen benachbarten konvexen Wandabschnitt übergeht.

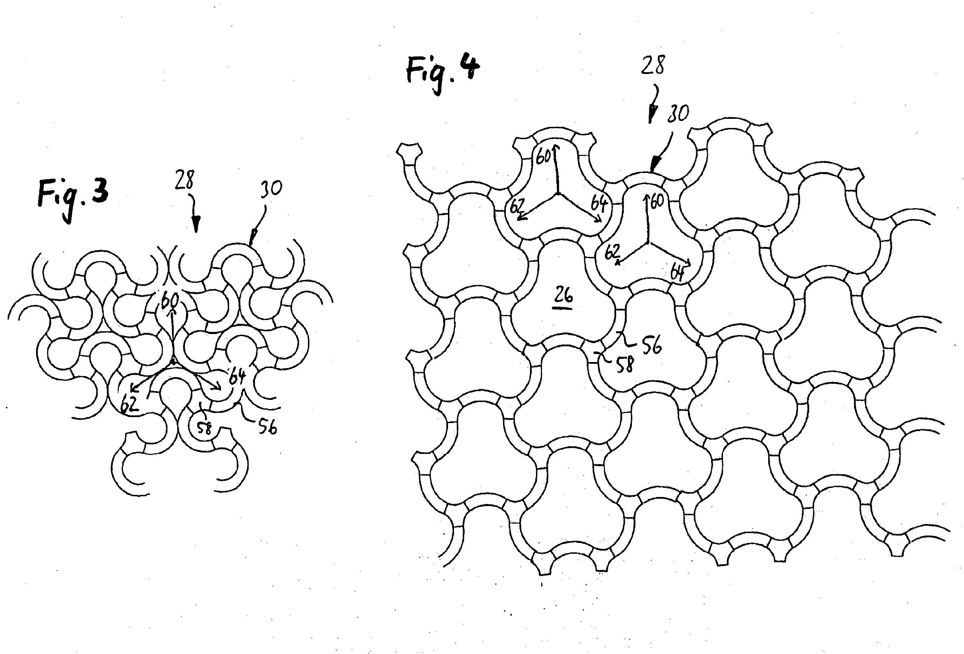

In Fig. 3 und 4 ist veranschaulicht, wie eine biegsame Welle 28 aus ihrer komprimierten bzw. unverformten Stellung gemäß Fig. 2c in eine teilweise expandierte bzw. gebogene Struktur übergeführt wird. Beim Biegen der Welle bzw. abschnittsweisen Expandieren von Zellen der Welle entfalten sich die drei einzelnen Bereiche der Wand 30, die jeweils aus einem konvexen und den beiden benachbarten konkaven Wandabschnitten gebildet sind, nach außen in drei Richtungen 60, 62 und 64. Zwischen diesen Richtungen 60, 62 und 64 besteht jeweils ein Winkel von 120°.

Wie insbesondere in Fig. 4 veranschaulicht ist, expandiert ein Zellenbereich der derart gestalteten biegsamen Welle gleichmäßig in die Richtungen 60, 62 und 64, wodurch die oben im Zusammenhang mit der Erfindung genannten Vorteile erzielt werden.

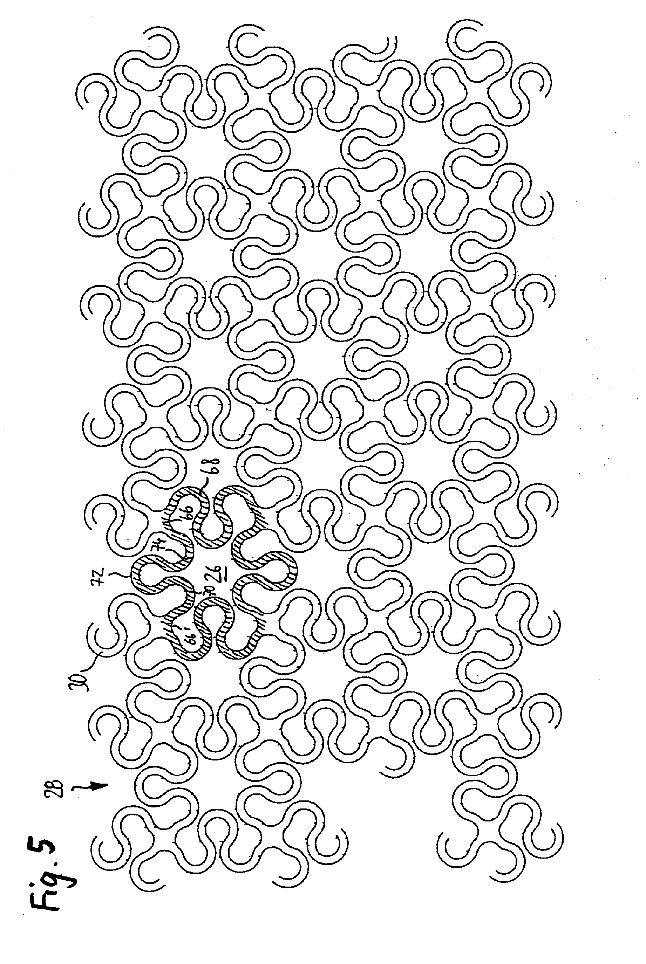

In Fig. 5 ist ein zweites Ausführungsbeispiel einer biegsamen Welle 28 veranschaulicht, bei der zwischen Knoten bzw. Verbindungselementen 66 mehrfach gefaltete Wandbereiche 68 ausgebildet sind. Die mehrfach gefalteten Wandbereiche 68 sind je einzeln aus drei im wesentlichen halbkreisförmigen Bögen 70, 72 und 74 gestaltet, die in der bezeichneten Zelle 26 aufeinanderfolgend einen konvexen, einen konkaven und einen weiteren konvexen Wandabschnitt bilden. In einer benachbarten Zelle bilden diese Bögen zugleich einen konkaven, einen konvexen und einen weiteren konkaven Wandabschnitt.

Die einzelnen Bögen 70, 72 und 74 überspannen einen Winkel von etwa 150° bis 250°, bevorzugt von zirka 200°. Ihre Oberflächen entsprechen im wesentlichen einem Abschnitt eines Kreiszylinders. Die Dicke der Wand der Bögen 70, 72 und 74 ist im wesentlichen konstant.

Die Knoten 66 weisen vier Seiten bzw. Endbereiche auf, an denen jeweils ein Wandabschnitt 68 anschließt. Zwischen diesen Endbereichen befinden sich konkave Rundungen 66', deren Radius jeweils dem inneren Radius eines Bogens 70, 72 oder 74 entspricht. Ein einzelner Knoten 66 ist somit im Querschnitt betrachtet im wesentlichen kreuzförmig gestaltet. Der Übergang zwischen einem Knoten 66 und einem Bogen 70, 72 oder 74 ist fließend bzw. glatt und ohne eine Kante gestaltet. Die Krümmung der Oberfläche am Übergang ist wiederum im wesentlichen konstant. Die einzelnen konkaven Rundungen 66' an den Knoten 66 erstrecken sich jeweils über einen Winkel von ca. 70° bis 110°, bevorzugt über einen Winkel von etwa 90°.

Auch bei dem Ausführungsbeispiel gemäß Fig. 5 ist die Wand 30 einer Zelle 26 der biegsamen Welle 28 demnach sämtlich aus abwechselnd konkaven und konvexen Wandabschnitten ausgebildet, die im Querschnitt betrachtet einzeln jeweils der Grundform eines Kreisbogens bzw. eines Kreisrings folgen. Von einem Knoten 66 stehen jeweils vier solcher Wandbereiche 68 ab. Die Struktur der biegsamen Welle gemäß Fig. 5 führt zu einer gleichzeitigen Expansion bevorzugt in vier Richtungen. Denkbar sind jedoch auch biegsame Wellen mit einer "spinnenartigen" Gestalt, in denen zumindest teilweise Zellen vorgesehen sind, die sämtlich aus abwechselnd konkaven und konvexen Wandabschnitten ausgebildet und deren Knoten fünft oder mehr Wandbereiche abstehen.

Die Herstellung der biegsamen Welle gemäß der Erfindung oder einer bevorzugten Ausführungsform hiervon kann sowohl aus Rohmaterial als auch aus Flachmaterial erfolgen, wobei bei letzterem die biegsame Welle später gerollt, geschweißt und/oder endbearbeitet wird. Weiterhin kann die Herstellung der biegsamen Welle mittels Laserschneiden, Laserabtrag, photochemisches Ätzen und/oder Erodieren erfolgen. Weiterhin kann die Herstellung der biegsamen Welle auch derart erfolgen, daß die biegsame Wellenstruktur in einer zumindest teilweise expandierten Form gefertigt wird und die biegsame Welle anschließend auf eine komprimierte Form verkleinert wird, bevor sie nachfolgend beim Einführen in einen Körper gebogen und dabei wieder zumindest teilweise expandiert wird.

Gemäß einer bevorzugten Ausführungsform der Erfindung wird die biegsame Welle zum Anordnen bzw. Einführen von einer Spreizstruktur in ein Hohlorgan verwendet, welche Spreizstäbe aufweist, die sich ausgehend von einem ersten Verbindungsabschnitt im Wesentlichen in einer Längsrichtung der Spreizstruktur bis zu einem zweiten Verbindungsabschnitt erstrecken, über den Umfang der Spreizstruktur verteilt angeordnet sind und durch radiales Aufspreizen an eine Wand des Hohlorgans anlegbar sind.

Spreizstrukturen der oben genannten Art können bevorzugt mit Hilfe der biegsamen Welle als Implantate für einen begrenzten Zeitraum oder auf Dauer in ein Hohlorgan, wie beispielsweise Herz, Blutgefäße, Gallenwege, ableitende Harnwege, Magen-Darm-Trakt, Uterus und Hirnventrikel, eingesetzt werden, um beispielsweise Thromben oder Gallengangssteine zurückzuhalten. So sind verschiedene Arten von Thrombosefilter bekannt, die als Venenimplantat perkutan über die Vena femoralis oder die Vena jugularis in die Vena cava inferior oder die Vena cava superior eingeführt werden. Dort soll das Venenimplantat Thromben auf ihrem Weg zum Herzen zurückhalten und dadurch einer Lungenembolie vorbeugen. Solche Implantate sind in der Regel mit konisch verlaufenden Streben oder Stäben gestaltet, die einen trichterförmigen Filterkorb bilden. Weiterhin kann die biegsame Welle zum Anordnen einer Metallelektrode verwendet werden, um an einer Wand des Hohlorgans Wärme einzubringen und dort beispielsweise eine Elektrokoagulation durchzuführen.

Ferner kann die biegsame Welle vorteilhaft zum Anordnen bzw. Einsezen von (bevorzugt selbstexpandierende) Endoprothesen zum Offenhalten von gangartigen Strukturen verwendet werden, die als sogenannte Stents auch in Blutgefäßen zum Einsatz kommen. Stents sind in der Regel mit einem schlauchförmigen, mehr oder weniger feinmaschigen Drahtgewebe oder Drahtgeflecht gestaltet, das unter einer elastischen Rückstellkraft seiner Drähte radial expandierbar ist.

Für derartige Verwendungen ist die biegsame Welle vorteilhaft im wesentlichen rohrförmig ausgestaltet und im Inneren kann eine ebenfalls biegsame Spreizwelle bzw. -stab bzw. -rohr angeordnet sein, mittels derer bzw. deren die Spreizstruktur bzw. die Metallelektrode bzw. der Stent manipuliert (z.B. expandiert und/oder kontrahiert bzw. im Außenumfang verkleinert werden kann), insbesondere mittels relativer Verschiebung bzw. Verlagerung der Spreizwelle gegenüber der rohrförmigen Welle.

### Bezugszeichenliste

- 26: Zelle
- 28: biegsame Welle
- 30: Wand
- 32: Kreisring
- 34: Kreisring
- 36: Kreisring
- 38: Kreisring
- 40: Kreisring
- 42: Kreisring
- 44: konkaver Wandabschnitt
- 46: konkaver Wandabschnitt
- 48: konkaver Wandabschnitt
- 50: konvexer Wandabschnitt
- 52: konvexer Wandabschnitt
- 54: konvexer Wandabschnitt
- 56: Kreisbogen
- 58: Knoten
- 58': Rundung
- 60: erste Richtung
- 62: zweite Richtung
- 64: dritte Richtung
- 66: Knoten
- 66': Rundung
- 68: gefalteter Wandbereich
- 70: Bogen
- 72: Bogen
- 74: Bogen

## Patentansprüche

1. Biegsame Welle (28) zum Einführen von medizinischen Produkten wie Stents in einen Kanal eines lebenden Körpers, insbesondere intraluminale, biegsame Welle, mit einer Vielzahl von aneinander angrenzenden Zellen (26), deren Wände (30) je aus Wandabschnitten ausgebildet sind, wobei bei mindestens einer Zelle (26) im Querschnitt betrachtet die Wand (30) in Umfangsrichtung abwechselnd aus jeweils einem konkaven Wandabschnitt (44, 46, 48) und einem benachbarten konvexen Wandabschnitt (50, 52, 54) ausgebildet ist, so dass eine zumindest lokale Biegung bzw. Verformung der biegsamen Welle möglich ist.

2. Biegsame Welle nach Anspruch 1,
**dadurch gekennzeichnet, daß** im Querschnitt betrachtet mindestens ein konvexer Wandabschnitt (50, 52, 54) mit einem Krümmungsradius ausgebildet ist, der im wesentlichen gleich einem Krümmungsradius eines benachbarten konkaven Wandabschnitts (44, 46, 48) gestaltet ist.

3. Biegsame Welle nach Anspruch 1 oder 2,
**dadurch gekennzeichnet, daß** im Querschnitt betrachtet die Wandabschnitte (44, 46, 48, 50, 52, 54) jeweils gleichartig oder fast gleichartig ausgebildet sind.

4. Biegsame Welle nach einem der Ansprüche 1 bis 3,
**dadurch gekennzeichnet, daß** im Querschnitt betrachtet die Wandabschnitt (44, 46, 48, 50, 52, 54) jeweils kreisbogenförmig ausgestaltet sind.

5. Biegsame Welle nach einem der Ansprüche 1 bis 4,
**dadurch gekennzeichnet, daß** im Querschnitt betrachtet mindestens eine Zelle (26) je aus mindestens drei konvexen Wandabschnitten (50, 52, 54) gestaltet ist, bei denen jeweils zwischen zwei konvexen Wandabschnitten (50, 52, 54) ein konkaver Wandabschnitt (44, 46, 48) ausgebildet ist.

6. Biegsame Welle nach einem der Ansprüche 1 bis 5,
**dadurch gekennzeichnet, daß** im Querschnitt betrachtet bei einem nicht verformten bzw. komprimierten Zustand der Zellen (26) der biegsamen Welle (28) die konvexen Wandabschnitte (50, 52, 54) mindestens einer Zelle (26) aneinaner anliegend ausgebildet sind.

7. Biegsame Welle nach einem der Ansprüche 1 bis 6,
**dadurch gekennzeichnet, daß** eine Gleitbeschichtung an der Oberfläche der biegsamen Welle angeordnet ist.

## Claims

1. A flexible shaft (28) for inserting medical products, such as stents, into a channel of a living body, in particular an intraluminal shaft, comprising a plurality of adjacent cells (26), the walls (30) of which each being formed as wall portions, wherein for at least one cell (26), when seen in cross-section, the wall (30) is alternately formed of a concave wall portion (44, 46, 48) and a neighbouring convex wall portion (50, 52, 54) in the circumferential direction, so that at least local bending or deformation of the flexible shaft is possible.

2. The flexible shaft according to claim 1, **characterized in that**, when seen in cross-section, at least one convex wall portion (50, 52, 54) is formed with a curvature radius that is substantially equal to a curvature radius of a neighbouring convex wall portion (44, 46, 48).

3. The flexible shaft according to claim 1 or 2, **characterized in that**, when seen in cross-section, the wall portions (44, 6, 48, 50, 52, 54) are each formed to be similar or almost similar.

4. The flexible shaft according to one of claims 1 to 3, **characterized in that**, when seen in cross-section, the wall portions (44, 6, 48, 50, 52, 54) are each designed in the form of a circular arc.

5. The flexible shaft according to one of claims 1 to 4, **characterized in that**, when seen in cross-section, at least one cell (26) is formed of at least three convex wall portions (50, 52, 54) in which one concave wall portion (44, 46, 48) is formed between two convex wall portions (50, 52, 54).

6. The flexible shaft according to one of claims 1 to 5, **characterized in that**, when seen in cross-section, in a non-deformed or compressed state of the cells (26) of the flexible shaft (28), the convex wall portions (50, 52, 54) of at least one cell (26) are formed to be adjacent to each other.

7. The flexible shaft according to one of claims 1 to 6, **characterized in that** a sliding coating is disposed on the surface of the flexible shaft.

## Revendications

1. Arbre flexible (28) pour l'introduction de produits médicaux comme des endoprothèses vasculaires dans un canal d'un corps vivant, notamment arbre flexible intracavitaire, avec une pluralité de cellules adjacentes les unes aux autres (26) dont les parois (30) sont chacune réalisées à partir de sections de paroi, dans lequel la paroi (30) est réalisée dans le sens périphérique en alternance à chaque fois à partir d'une section de paroi concave (44, 46, 48) et d'une section de paroi convexe voisine (50, 52, 54) pour au moins une cellule (26) vue en coupe transversale de sorte qu'une flexion ou déformation au moins locale de l'arbre flexible est possible.

2. Arbre flexible selon la revendication 1,
**caractérisé en ce que**, vue en coupe transversale, au moins une section de paroi convexe (50, 52, 54) est réalisée avec un rayon de courbure qui est formé de manière essentiellement identique à un rayon de courbure d'une section de paroi concave voisine (44, 46, 48).

3. Arbre flexible selon la revendication 1 ou 2,
**caractérisé en ce que**, vues en coupe transversale, les sections de paroi (44, 46, 48, 50, 52, 54) sont à chaque fois réalisées de manière similaire ou presque similaire.

4. Arbre flexible selon une des revendications 1 à 3,
**caractérisé en ce que**, vues en coupe transversale, les sections de paroi (44, 46, 48, 50, 52, 54) sont chacune configurées en forme d'arc de cercle.

5. Arbre flexible selon une des revendications 1 à 4,
**caractérisé en ce que**, vue en coupe transversale, au moins une cellule (26) est à chaque fois formée d'au moins trois sections de paroi convexes (50, 52, 54) pour lesquelles une section de paroi concave (44, 46, 48) est à chaque fois réalisée entre deux sections de paroi convexes (50, 52, 54).

6. Arbre flexible selon une des revendications 1 à 5,
**caractérisé en ce que**, vues en coupe transversale, dans le cas d'un état non déformé ou comprimé des cellules (26) de l'arbre flexible (28), les sections de paroi convexes (50, 52, 54) d'au moins une cellule (26) sont réalisées de manière tangente les unes aux autres.

7. Arbre flexible selon une des revendications 1 à 6,
**caractérisé en ce qu'**un revêtement glissant est disposé à la surface de l'arbre flexible.
